# FASCICULE DE BREVET EUROPEEN

(11) **EP 0 658 609 B1**
(45) Date de publication et mention de la délivrance du brevet: **12.12.2001**
(21) Numéro de dépôt: 94402480.1
(22) Date de dépôt: 03.11.1994
(51) Int. Cl.: C09D 201/00, A61K 7/043

(54) **Composition filmogène contenant un copolymère d'alkyle fluoré, utilisable comme vernis-à-ongles**
Filmbildende Zusammensetzung, welche ein Copolymer eines fluoriniertes Alkyl enthält, verwendbar als Nagellack
Film-forming composition containing a copolymer of a fluorinated alkyl, useful as nail varnish

(30) Priorité: 15.12.1993 FR 9315069
(43) Date de publication de la demande: 21.06.1995
(73) Titulaire: L'OREAL, 75008 Paris (FR)
(72) Inventeur: De La Porterie, Valérie, F-77820 Le Chatelet en Brie (FR)
(74) Mandataire: Dodin, Catherine

(56) Documents cités:
- EP-A- 0 195 714
- EP-A- 0 473 148
- EP-A- 0 554 667
- EP-A- 0 558 423
- FR-A- 2 282 857
- DATABASE WPI Week 9237, Derwent Publications Ltd., London, GB; AN 304635 & JP-A-4 210 613 (KAO CORP) 31 Juillet 1992 & PATENT ABSTRACTS OF JAPAN vol. 16, no. 552 (C-1006)
- PATENT ABSTRACTS OF JAPAN vol. 17, no. 533 (C-1114) 27 Septembre 1993 & JP-A-05 148 122 (KAO CORP.) 15 Juin 1993 & DATABASE WPI Week 9328, Derwent Publications Ltd., London, GB; AN 224246

## Description

L'invention a pour objet une composition filmogène contenant un copolymère d'alkyle fluoré utilisable notamment dans le domaine cosmétique pour le maquillage et/ou le soin du visage et/ou des cheveux. En particulier, la composition de l'invention peut être utilisée comme eye-liner, mascara, ou laque et plus spécialement comme vernis-à-ongles, incolore ou coloré.

De façon plus précise, l'invention se rapporte à une composition à milieu aqueux contenant des polymères filmogènes, capable de former sur un support (cil, cheveu, ongle) un film homogène et continu.

Les vernis-à-ongles à l'eau sont actuellement des produits de maquillage en plein développement en vue de se substituer aux vernis à solvants, non seulement pour des raisons de sécurité pour la consommatrice, mais aussi pour des raisons d'environnement du fait de l'absence de composés volatils dans de telles formulations.

Ces récents produits de maquillage doivent néanmoins présenter des caractéristiques cosmétiques de qualité équivalente à celles des vernis-à-ongles à solvants, connus de l'homme de l'art comme une application facile, un séchage du film rapide, l'obtention d'un film brillant adhérent sur la surface de l'ongle, une bonne tenue dans le temps et plus particulièrement une bonne résistance à l'eau.

En outre, ces produits doivent être non irritants pour la peau et les ongles.

Or, les vernis à l'eau présentent souvent l'inconvénient d'une mauvaise tenue dans le temps due essentiellement à une mauvaise résistance à l'eau, soit parce que le film peut se redissoudre dans l'eau, soit parce que le film se ternit en présence d'eau, soit parce que le film blanchit sous l'action de l'eau, soit encore parce que le film se décolle dans l'eau.

Pour éviter ces problèmes de résistance à l'eau, on peut envisager, soit l'utilisation de polymères qui ne sont pas susceptibles de se dissoudre dans l'eau, mais cela n'est parfois pas suffisant pour éviter les autres inconvénients cités ci-dessus, soit l'utilisation d'additifs qui vont empêcher l'eau de pénétrer et de faire décoller le film en la repoussant ou la faisant glisser sur le film. Ces additifs joueraient le rôle de protecteur du film de vernis vis-à-vis de l'eau.

Dans le domaine capillaire, il est connu du document FR-A-2385391 des compositions aqueuses de lavage contenant des copolymères d'alkyle fluoré issus d'une copolymérisation d'un monomère acrylique fluoré et d'un monomère acrylique aminé. Ces copolymères insolubles dans l'eau sont mis en suspension dans l'eau à l'aide de tensioactifs. Ils ont pour but d'améliorer le temps de séchage des cheveux traités.

En revanche, ces compositions ne permettent absolument pas la formation d'un film homogène et continu.
Des copolymères d'alkyle fluoré issus d'une copolymérisation d'un monomère acrylique fluoré et d'un monomère acrylique à radical alkyle, hydroxyalkylène ou alkylaminé, sont décrits dans le document WO-A-92/16103 pour protéger de l'eau ou de l'huile des substances actives présentes dans les herbicides, insecticides ou pesticides, en vue d'augmenter leur efficacité. Ces copolymères sont dispersés dans de l'eau via des tensioactifs.

Comme précédemment, ces compositions ne permettent pas la formation de films homogènes et continus, destinés en particulier au domaine cosmétique.

Par ailleurs, le document EP-A-0206671 divulgue l'emploi de copolymères d'alkyle fluoré solubilisés dans une huile volatile ou un solvant organique, dans des rouges-à-lèvres, mascaras, eye-liners, et vernis-à-ongles. Ces produits cosmétiques présentent l'inconvénient de contenir une forte proportion d'huile et/ou de solvant organique pour solubiliser ces polymères, néfastes pour l'environnement.

L'invention a justement pour objet une nouvelle composition filmogène en milieu aqueux, utilisable en cosmétique, permettant de remédier aux inconvénients cités ci-dessus.

Cette composition présente en particulier une bonne résistance à l'eau et une bonne tenue dans le temps tout en conservant de bonnes propriétés cosmétiques, et une grande aptitude à former des films homogènes et continus.

De façon surprenante, les inventeurs ont trouvé que l'incorporation d'un copolymère d'alkyle fluoré en émulsion, dans une composition cosmétique aqueuse, conférait à cette dernière une bonne résistance à l'eau.

Plus précisément, l'invention a pour objet une composition cosmétique filmogène selon la revendication 1.

Par "fluoré", on doit comprendre une substitution totale ou partielle des atomes d'hydrogène de la chaîne alkyle par des atomes de fluor. De préférence, la substitution est totale.

Par "radical hydroxycarboné", il faut comprendre un radical hydroxyalkyle ou hydroxyalkylène.

Les copolymères de l'invention confèrent, contre toute attente, et malgré la non dissolution de ces copolymères dans l'eau, une bonne filmification de la composition. En effet, les processus de filmification en milieu aqueux et en milieu solvant organique (par exemple dans le cas des vernis-à-ongles) sont totalement différents. En milieu aqueux, on part de particules de polymères distinctes qui se lient puis interdiffusent au fur et à mesure que l'eau s'évapore du support alors qu'en milieu solvant, les chaînes polymériques sont liées entre elles et enchevêtrées au départ, avant même d'appliquer la composition (vernis) sur le support.

Ainsi, le copolymère fluoré aurait pu nuire à la filmification de la composition, conduisant notamment à une fragilisation et une mauvaise cohésion du film, le rendant inutilisable.

En outre, les films obtenus présentent une résistance à l'eau améliorée par rapport à ceux résultant des compositions filmogènes de l'art antérieur, exemptes de copolymères fluorés.

La composition de l'invention peut contenir moins de 30% en poids de solvant organique miscible à l'eau et de préférence de 0% à 15%. Ce taux faible voire nul de solvant confère à la composition, lors de son utilisation dans le domaine cosmétique, une meilleure innocuité vis-à-vis du support.

Comme solvant utilisable dans l'invention, on peut citer des alcools en C₁ à C₆ (éthanol, isopropanol), des cétones (acétone, méthyl-éthyl cétone), des acétates comme l'acétate de butyle, d'éthyle, des hydrocarbures saturés en C₅ à C₁₂ comme l'hexane ou l'octane.

De façon avantageuse, le premier monomère fluoré est un monomère de type acrylique présentant en particulier la formule (B) suivante : 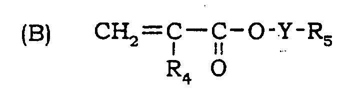 où R₄ représente un atome d'hydrogène ou un radical alkyle en C₁ à C₄ ; Y représente un radical alkylène en C₁ à C₆ et R₅ représente un radical alkyle en C₁ à C₂₀ dont tout ou partie des atomes d'hydrogène est remplacé par des atomes de fluor.

Selon l'invention, les radicaux alkyles, fluorés ou non, et les radicaux alkylénés peuvent être linéaires ou ramifiés.

A titre d'exemples, le premier monomère fluoré peut présenter la formule: 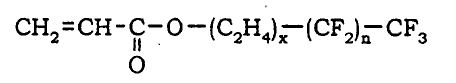
avec n allant de 4 à 16 et x valant 1 ou 2 ;
ceci correspond à : R₄ = H; Y= - C₂H₄- ou -C₄H₈ - et R₅ = -(CF₂)ₙ - CF₃ dans la formule (B).
En particulier, n vaut 5, 7, 9, 11.

Le premier monomère fluoré peut aussi présenter la formule: 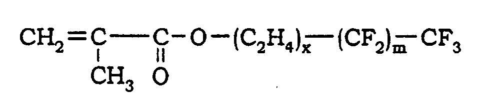
avec m allant de 4 à 16 et x valant 1 ou 2 ;
ceci correspond à : R₄ = - CH₃ ; Y = - C₂H₄ - ou - C₄H₈ - et R₅ = - (CF₂)ₘ - CF₃
En particulier, m vaut 5, 7, 9, 11.

Comme monomère fluoré utilisable de l'invention, on peut encore citer les composés du type : 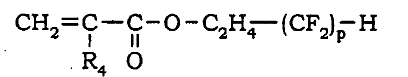 avec R₄ = H ou - CH₃ et p allant de 6 à 16 et par exemple égal à 6 ou 8.

A titre d'exemple préféré, le second monomère est tel que R₁ représente un hydrogène ou un radical alkyle en C₁ à C₄ et par exemple un radical méthyle, éthyle, n-butyle ou isopropyle.

En outre, le rapport en poids de premier monomère fluoré par rapport au second monomère non fluoré peut aller de 10/90 à 90/10 et par exemple de 12/88 à 50/50.

En particulier, les copolymères fluorés de l'invention peuvent être fabriqués comme décrits dans les documents FR-A-2175332, FR-A-2540131 et EP-A-206671.

Ces copolymères sont par exemple ceux vendus par ATOCHEM sous la marque Foraperle (R) 344. On peut aussi utiliser les Foraperle (R) 145, 333, 390, 350 qui sont des monomères fluorés aminés.

Ces polymères fluorés se présentent sous forme de dispersion dans l'eau contenant éventuellement un alcool et/ou une cétone en faible quantité, dispersion que l'on peut diluer en toute proportion dans l'eau.

Les copolymères selon l'invention peuvent être introduits dans toutes compositions aqueuses à base de dispersions aqueuses de matières filmogènes, en particulier de polymères filmogènes tels que des dispersions de polyuréthannes, de polymères acryliques, vinyliques, styrène-acryliques ou toute autre dispersion aqueuse de polymères ou copolymères compatibles avec les copolymères fluorés selon l'invention en milieu aqueux.

Les matières filmogènes utilisables dans l'invention sont par exemple les polymères vendus sous forme de dispersion en solution aqueuse par la Société SANNCOR et notamment le Sancure 878 qui est un polyuréthanne-polyéther dispersé dans de l'eau.

Ces matières filmogènes peuvent être utilisées à raison de 2% à 60%, et de préférence de 4% à 30% en poids de matière active par rapport au poids total de la composition.

Les copolymères fluorés de l'invention peuvent être utilisés comme additifs. Ils représentent en particulier de 0,005% à 10% en poids de matière active par rappôrt au poids total de la composition, et de préférence de 0,5% à 5%. Ces proportions sont parfaitement bien adaptées à la réalisation d'un vernis-à-ongles à base aqueuse.

Aussi, l'invention a encore pour objet un vernis-à-ongles à base aqueuse consistant en une composition telle que définie précédemment.

En dehors des vernis-à-ongles, la composition de l'invention peut constituer un mascara, un eye-liner, ou encore une laque à l'eau et de façon plus générale toute composition cosmétique filmogène devant résister à l'eau.

La composition de l'invention permet l'obtention d'un extrait sec pouvant aller de 20% à 60% et de préférence de 25% à 45 %.

Par ailleurs, la composition de l'invention peut être incolore ou colorée. Dans ce dernier cas, on utilise au moins un pigment de nature organique ou inorganique ou un colorant organique.

Ces matières colorantes, lorsqu'elles existent, sont généralement utilisées à raison de 0,01% à 25% et mieux, de 0,5% à 20% en poids par rapport au poids total de la composition.

Parmi les pigments organiques utilisables dans l'invention, on peut citer les D&C Red n° 5, n° 6, n° 7, n° 10, n° 11, n° 12, n° 13, n° 34, les laques D&C Yellow n° 5 et D&C Red n° 2, ainsi que la guanine.

Parmi les pigments inorganiques utilisables, on peut citer le dioxyde de titane, l'oxyde de fer brun, l'oxyde de fer noir, les oxydes de fer rouge et l'oxychlorure de bismuth.

La composition de l'invention peut, en outre, contenir des adjuvants à raison de 0,01% à 10% en poids par rapport au poids total de la composition et de préférence moins de 5%. La quantité précise d'additif est fonction de l'adjuvant utilisé et est facilement déterminable par l'homme du métier.

Ces adjuvants sont notamment ceux classiquement utilisés dans le domaine cosmétique comme les filtres UV, les conservateurs, les agents mouillants, dispersants ou antimousses, les cires, les accélérateurs de séchage ou d'étalement, ou encore des composés améliorant la brillance ou la dureté du film obtenu.

Les compositions selon l'invention peuvent contenir, en outre, au moins un agent épaississant à une proportion allant de 0,01% à 5% et de préférence de 0,1% à 1%, en poids par rapport au poids total de la composition.

Parmi les agents épaississants appropriés pour la formulation de ces compositions filmogènes aqueuses, on peut citer la cellulose et ses dérivés telles que la carboxyméthylcellulose et l'hydroxyéthylcellulose, les silicates, les argiles telles que la laponite, les polymères synthétiques tels que les polymères acryliques ou les polymères associatifs de type polyuréthanne ou hydroxyéthylcellulose modifiés par une chaîne hydrophobe, et les gommes naturelles telles que la gomme de carraghénane ou de xanthane.

On utilise de préférence un agent épaississant choisi parmi l'hydroxyéthylcellulose, la laponite, et les polyuréthannes associatifs.

Par ailleurs, la composition de l'invention peut contenir un agent tensioactif pour émulsionner les copolymères dans l'eau, et donc favoriser leur dispersion.

L'agent tensioactif utilisable dans l'invention présente notamment de préférence une valeur de HLB (équilibre lipophile/hydrophile) ≥ 10. Il peut être constitué par n'importe quel agent hydrophile cosmétiquement acceptable, commercialisé et notamment par un composé non ionique polyéthoxylé de HLB = 14 éventuellement associé à un ammonium quaternaire à chaîne grasse.

En général, on utilise de 0% à 10% en poids d'agent tensioactif par rapport au poids total de la composition et de préférence de 3% à 5%.

La description qui suit, donnée à titre illustratif et non limitatif, permet de mieux faire ressortir l'invention ainsi que ses caractéristiques et avantages.

L'invention étant parfaitement bien adaptée à la réalisation d'un vernis-à-ongles, les exemples 1 à 3 qui suivent porteront sur des vernis, bien que d'autres applications cosmétiques puissent être envisagées comme indiqué précédemment. Les exemples 5 et 6 illustrent deux autres type d'application cosmétique.

Les compositions ci-après ont été obtenues sous agitation, en dispersant les copolymères fluorés (Foraperle) dans de l'eau, contenant éventuellement un conservateur et un solvant miscible à l'eau (alcool) et en ajoutant à la dispersion les matières filmogènes, éventuellement les épaississants, les cires, puis les pigments.

Dans les exemples, les proportions sont données en % pondéral.

### . Exemple 1 : Composition de vernis à ongles (témoin)

- Sancure 878 (37,5%)* 75,0
- DC Red 30 0,5
- TiO₂ 0,5
- Epaississant polyuréthanne associatif (SER AD Fx 1100 de la société SERVO) 0,3
- Conservateur 0,04
- Eau qsp 100
(*) Les chiffres entre parenthèses indiquent la proportion de matière active.

L'application de ce vernis à ongles est aisée et son temps de séchage est court. Le film obtenu après séchage est continu, brillant et homogène.

### . Exemple 2 : Composition de vernis à ongles

- Sancure 878 (39%)* 70,00
- SER AD Fx 1100 0,3
- DC Red 30 0,5
- Conservateur 0,02
- Foraperle 145 (25%)* 4,00
- Eau qsp 100

Le vernis-à-ongles de l'exemple 2 présente les mêmes propriétés que celles de l'exemple 1. L'addition de Foraperle conserve donc les propriétés cosmétiques du vernis. De plus, on observe une amélioration de la résistance à l'eau du vernis.

### . Exemple 3 : Composition de vernis à ongles

- Sancure 878 (39%)* 72,0
- SER AD Fx 1100 0,08
- DC Red 30 0,5
- Conservateur 0,02
- Foraperle 333 (28%)* 3,6
- isopropanol 13,8
- Eau qsp 100

Le vernis-à-ongles de l'exemple 3 présente des propriétés équivalentes à celles du vernis-à-ongles de l'exemple 2. La présence d'une petite quantité d'alcool améliore son élimination.

### . Exemple 4 : Composition de vernis à ongles

- Sancure 878 (37,2%)* 72,6
- Foraperle 145 (25%)* 22,4
- SER AD FX 1100 0,08
- Pigments DC Red 30 0,5
- Conservateurs 0,02
- Eau qsp 100

Le vernis-à-ongles de l'exemple 4 présente des propriétés équivalentes à celles des vernis-à-ongles des exemples 2 et 3.

### . Exemple 5 : Influence de la proportion de polymère fluoré dans la composition.

Dans le tableau I, ci-après, on donne les propriété des films obtenus à partir des compositions des exemples 2, 4 et du contre-exemple suivant :

### Contre-Exemple :

- Sancure 878 (37,2%).* /
- Foraperle 145 (25%) * 80,0
- SER AD FX 1100 0,25
- Pigments DC Red 30 1,0
- Conservateurs 0,05
- Eau qsp 100

**TABLEAU I**

| Exemple | 2 | 4 | Contre-exemple |
|---|---|---|---|
| Extrait Sec (%) | 28,5 | 33 | 22 |
| % Final Foraperle | 1 | 5,6 | 20 |
| Brillance du film | 81,5 | 52,4 | 68,1 |
| Résistance à l'eau | > 3 H 30 | > 4 H | <2H45 |

La brillance correspond au coefficient de réflexion lumineux du film. La détermination de la résistance à l'eau sera explicitée ultérieurement.

D'après le tableau I, on observe que dans les compositions de l'invention, le Foraperle améliore bien la résistance à l'eau à 1% ou 5% mais diminue sensiblement la brillance du film à 5%, ce qui n'est pas souhaité. Un film doit présenter, pour être satisfaisant, une brillance ≥ 75.

Lorsque le Foraperle est utilisé seul (contre-exemple), la brillance du film obtenu n'est pas bonne (plutôt film mat) ; sa résistance à l'eau est moyenne et sa couvrance nulle (au moins 3 couches sont nécessaires).

Le Foraperle peut donc bien être utilisé comme additif, en association avec une autre dispersion aqueuse filmogène, à un pourcentage de préférence <5%.

### . Exemple 6 : Résistance à l'eau du vernis

On donne ci-après des tests comparatifs relatifs à des bases de vernis-à-ongles à l'eau, conformes à l'invention et à l'art antérieur.

La résistance à l'eau des films obtenus peut être contrôlée de 2 façons différentes :
- soit en observant le comportement de chaque film obtenu sur kératine, sous agitation dans l'eau chaude (45°C) + tensioactif ;
- soit en déposant une goutte d'eau sur chaque film obtenu et en mesurant l'angle que forme cette goutte avec le film de vernis. Plus l'angle est élevé, plus l'eau est repoussée par la surface sur laquelle elle est déposée.

Ces 2 tests ont été réalisés sur des vernis-à-ongles à l'eau à base d'une dispersion de polyuréthanne (Sancure 878) contenant un copolymère fluoré, conformément à l'invention et ne contenant pas de copolymère fluoré, conformément à l'art antérieur.

### Test de résistance du film dans l'eau

Le vernis est appliqué sur un morceau de kératine qui est laissé à un séchage à humidité et température constantes pendant 1 heure. Puis le morceau de kératine est agité dans l'eau chaude (45°C) en présence de détergent (1% Teepol ^{(R)}). On observe alors le comportement du film : blanchissement, décoloration, solubilisation, décollement en fonction du temps.

Les résultats sont portés dans le tableau II ci-après :

**TABLEAU II**

| **Essai** | **Résultat obtenu** |
|---|---|
| - sans copolymère fluoré : exemple 1 | - décollement du film avant 8 mn |
| - avec 1% M.A.* Foraperle 145: exemple 2 | - léger blanchissement, pas de décollement après 3 heures. |
| - avec 1 % M.A.* Foraperle 333: exemple 3 | - pas de blanchissement, pas de décollement après 3 heures. |

| | |
|---|---|
| (*) M.A. = Matière active. | |

On observe bien, d'après le tableau II, que les essais avec les copolymères selon l'invention résistent mieux à l'eau que l'essai sans copolymère.

### Test de la goutte d'eau

On mesure l'angle que forme une goutte d'eau avec la surface du film de vernis obtenu pour chaque essai testé.

Les résultats sont portés dans le tableau III, ci-après:

On observe bien, d'après le tableau III, que les angles obtenus avec les essais selon l'invention sont plus grands que ceux de l'essai sans copolymère. Ceci montre que les films obtenus selon l'invention repoussent davantage l'eau que l'essai sans copolymère.

**TABLEAU III**

| **Essai** | **Angle avec l'eau en degré** |
|---|---|
| - sans copolymère : exemple 1 | 69,3 |
| - avec 1% M.A.* Foraperle 145 : exemple 2 | 73,0 |
| - avec 1% M.A.* Foraperle 333 : exemple 3 | 79,5 |

### . Exemple 7 : Composition "Mascara"

- Cire d'abeilles 10,0
- Cire de Carnauba 2,0
- Paraffine 7,0
- Stéarate de triéthanolamine 10,0
- Oxyde de fer noir 3,0
- Bleu d'outremer 1,5
- Gomme arabique 2,0
- Hydroxyéthylcellulose 1,0
- Panthénol 1,0
- Conservateurs 0,3
- Dispersion aqueuse filmogène (34%)* (SANCURE 2060) 15,0
- Foraperle 145 (25%)* 4,0
- Eau qsp 100

Le mascara appliqué sur les cils présente une bonne résistance à l'eau.

### . Exemple 8 : Composition "Eye-Liner"

- Alcool polyvinylique 2,0
- Propylène glycol 5,0
- Hydroxyéthylcellulose 0,1
- Laponite 0,2
- Laurylsulfate de sodium 0,1
- Oxyde de fer noir 15,0
- Dispersion aqueuse filmogène (34%)* (SANCURE 2060) 14,7
- Foraperle 145 (25%) * 4,0
- Eau qsp 100

L'eye-liner appliqué présente une bonne résistance à l'eau.

## Revendications

1. Composition cosmétique filmogène comprenant un milieu aqueux utilisable en cosmétique, **caractérisée en ce qu'**elle comprend, dispersés dans ledit milieu aqueux, au moins une matière filmogène et un copolymère fluoré résultant de la copolymérisation d'au moins un premier monomère fluoré vinylique de formule (B): 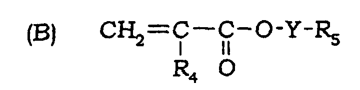 où R₄ représente un atome d'hydrogène ou un radical alkyle en C₁ à C₄ ; Y représente un radical alkylène en C₁ à C₆ et R₅ représente un radical alkyle en C₁ à C₂₀ dont tout ou partie des atomes d'hydrogène est remplacé par des atomes de fluor,
et d'au moins un second monomére de formule (A): 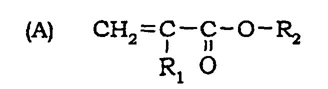
avec R₁ représentant un atome d'hydrogène ou un radical alkyle en C₁ à C₄ e R₂ représentant un radical alkyle en C₁ à C₆, un radical hydroxycarboné en C₂ à C₄ ou un radical -(CH₂)p-NH-R₃ avec R₃ représentant un alkyle en C₁ à C₆ ou un cycloalkyle et p étant un entier allant de 1 à 4,
le rapport en poids de premier monomère par rapport au second monomère nor fluoré allant de 10/90 à 90/10,
le milieu aqueux consistant en de l'eau, de 0 à 30 % en poids de solvant organique miscible à l'eau, et éventuellement une matière colorante, un adjuvant, un agent épaississant, et/ou un agent tensioactif.

2. Composition selon la revendication 1, **caractérisée en ce que** l'adjuvant est choisi parmi les filtres UV, les conservateurs, les agents mouillants, dispersants ou antimousse, les cires, les accélérateurs de séchage, les agents d'étalement, les composés améliorant la brillance ou la dureté du film obtenu après séchage de la composition.

3. Composition selon la revendication 1 ou 2, **caractérisée en ce qu'**elle est exempte de solvant organique.

4. Composition selon l'une quelconque des revendications précédentes, **caractérisée en ce que** R₂ est un radical alkyle en C₁ à C₆.

5. Composition selon l'une quelconque des revendications précédentes, **caractérisée en ce que** le premier monomère présente la formule : 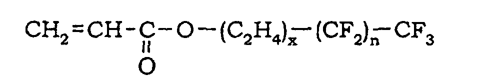 avec n allant de 4 à 16 et x valant 1 ou 2.

6. Composition selon l'une quelconque des revendications 1 à 4, **caractérisée en ce que** le premier monomère présente la formule: 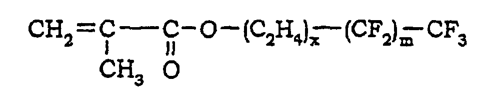 avec m allant de 4 à 16 et x valant 1 ou 2.

7. Composition selon l'une quelconque des revendications 1 à 4, **caractérisée en ce que** le premier monomère présente la formule : 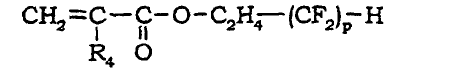 avec R₄ = H ou -CH₃ et p allant de 6 à 16 et par exemple égal à 6 où 8.

8. Composition selon l'une quelconque des revendications précédentes, **caractérisée en ce que** le copolymère fluoré représente de 0,005% à 10% en poids par rapport au poids total de la composition.

9. Composition selon l'une quelconque des revendications précédentes, **caractérisée en ce que** la matière filmogène est composée d'au moins un polymère filmogène.

10. Composition selon la revendication 9, **caractérisée en ce que** le polymère est choisi parmi les polyuréthannes, les polymères vinylique, les polymères acryliques, et les styrène-acryliques.

11. Composition selon l'une quelconque des revendications précédentes, **caractérisée en ce que** le polymère est choisi parmi les polyéther-polyuréthanes et les polyester-polyuréthanes.

12. Composition selon l'une quelconque des revendications précédentes, **caractérisée en ce qu'**elle contient un agent tensioactif pour émulsionner le copolymère fluoré.

13. Composition selon l'une quelconque des revendications précédentes, **caractérisée en ce que** la matière colorante est un colorant et/ou un pigment

14. Composition selon l'une quelconque des revendications précédentes, **caractérisée en ce qu'**elle contient, en poids :
- de 0,005% à 10% de copolymère fluoré,
- de 2% à 60% de matière filmogène,
- de 0% à 10% d'agent tensioactif,
- de 0% à 10% d'un adjuvant,
- de 0% à 25% de matière colorante,
- de 0% à 5% d'épaississant, et
- de l'eau en quantité suffisante pour faire 100%.

15. Composition selon l'une quelconque des revendications précédentes, **caractérisée en ce que** le copolymère fluoré représente de 0,5% à 5% en poids par rapport au poids total de la composition.

16. Composition selon l'une quelconque des revendications précédentes, **caractérisée en ce que** la matière filmogène représente de 4% à 30% en poids par rapport au poids total de la composition.

17. Composition selon l'une quelconque des revendications précédentes, **caractérisée en ce qu'**elle se présente sous la forme d'un mascaras, d'un eye-liner ou d'une laque.

18. Composition selon l'une quelconque des revendications 1 à 16, **caractérisée en ce qu'**elle se présente sous la forme d'un vemis-à-ongles.

19. Procédé de maquillage et/ou de soin de la peau, des ongles, des cils et/ou des cheveux, **caractérisé par le fait que** l'on applique sur la peau, les ongles, les cils et/ou les cheveux une composition telle que définie selon l'une quelconque des revendications 1 à 18.

20. Utilisation dans une composition cosmétique comprenant un milieu aqueux utilisable en cosmétique, d'au moins une matière filmogène et d'au moins un copolymère fluoré tels que définis selon l'une quelconque de revendications 1 à 11 et 15,16, dispersés dans ledit milieu aqueux, pour l'obtention d'un film homogène et continu et/ou résistant à l'eau et/ou ayant une bonne tenue dans le temps, le milieu aqueux ne contenant que de l'eau, de 0 à 30 % en poids de solvant organique miscible à l'eau, et éventuellement un ingrédient choisi parmi les matières colorantes, les adjuvants, les agents épaississants, les agents tensioactifs.

## Patentansprüche

1. Filmbildende kosmetische Zusammensetzung, die ein in der Kosmetik verwendbares wäßriges Medium enthält, **dadurch gekennzeichnet, daß** sie, dispergiert in dem wäßrigen Medium, mindestens ein filmbildendes Material und mindestens ein fluorhaltiges Copolymer enthält, das durch Copolymerisation mindestens eines ersten, fluorhaltigen Vinylmonomers der Formel (B) 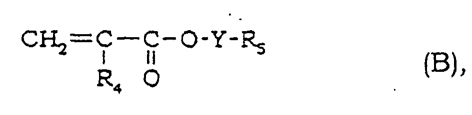 in der bedeuten:
- R₄ Wasserstoff oder C₁-C₄-Alkyl,
- Y einen C₁-C₆-Alkylenrest und
- R₅ C₁-C₂₀-Alkyl, worin alle Wasserstoffatome oder ein Teil der Wasserstoffatome durch Fluoratome ersetzt sind/ist,
und mindestens eines zweiten Monomers der Formel (A) 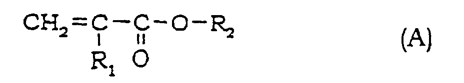 entsteht, in der bedeuten:
- R₁ Wasserstoff oder C₁-C₄-Alkyl und
- R₂ C₁-C₆-Alkyl, einen C₂-C₄-Hydroxykohlenstoff-Rest oder einen Rest -(CH₂)ₚ-NH-R₃, worin R₃ C₁-C₆-Alkyl oder Cycloalkyl darstellt und p eine ganze Zahl im Bereich von 1 bis 4 ist,
wobei das Gewichtsverhältnis des ersten Monomers zum zweiten, kein Fluor enthaltenden Monomer im Bereich von 10/90 bis 90/10 liegt und wobei das wäßrige Medium aus Wasser, 0 bis 30 Gew.-% organischem, mit Wasser mischbarem Lösemittel und gegebenenfalls einem Färbemittel, einem Hilfsstoff, einem Verdickungsmittel und/oder einem grenzflächenaktiven Stoff besteht.

2. Zusammensetzung nach Anspruch 1, **dadurch gekennzeichnet, daß** der Hilfsstoff unter UV-Filtern, Konservierungsmitteln, Netzmitteln, Dispergiermitteln, Antischaummitteln, Wachsen, Trocknungsbeschleunigern, Spreitmitteln, Verbindungen zur Verbesserung des Glanzes oder der Härte des Films, der nach der Trocknung der Zusammensetzung erhalten wird, ausgewählt ist.

3. Zusammensetzung nach Anspruch 1 oder 2, **dadurch gekennzeichnet, daß** sie kein organisches Lösemittel enthält.

4. Zusammensetzung nach einem der vorhergehenden Ansprüche, **dadurch gekennzeichnet, daß** es sich bei R₂ um einen C₁-C₆-Alkylrest handelt.

5. Zusammensetzung nach einem der vorhergehenden Ansprüche, **dadurch gekennzeichnet, daß** das erste Monomer die Formel 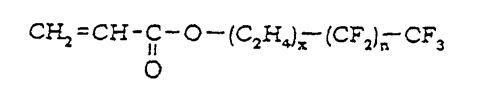 aufweist, worin n im Bereich von 4 bis 16 liegt und x die Zahl 1 oder 2 bedeutet.

6. Zusammensetzung nach einem der Ansprüche 1 bis 4, **dadurch gekennzeichnet, daß** das erste Monomer die Formel 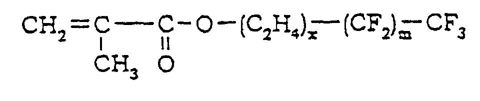 aufweist, worin m im Bereich von 4 bis 16 liegt und x die Zahl 1 oder 2 bedeutet.

7. Zusammensetzung nach einem der Ansprüche 1 bis 4, **dadurch gekennzeichnet, daß** das erste Monomer die Formel 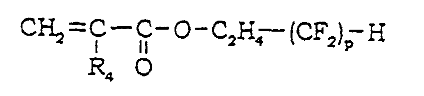 aufweist, worin R₄ Wasserstoff oder -CH₃ bedeutet und p im Bereich von 6 bis 16 liegt und beispielsweise gleich 6 oder 8 ist.

8. Zusammensetzung nach einem der vorhergehenden Ansprüche, **dadurch gekennzeichnet, daß** das fluorhaltige Copolymer in einem Mengenanteil von 0,005 bis 10 Gew.-%, bezogen auf das Gesamtgewicht der Zusammensetzung, enthalten ist.

9. Zusammensetzung nach einem der vorhergehenden Ansprüche, **dadurch gekennzeichnet, daß** das filmbildende Material aus mindestens einem filmbildenden Polymer zusammengesetzt ist.

10. Zusammensetzung nach Anspruch 9, **dadurch gekennzeichnet, daß** das Polymer unter Polyurethanen, Vinyl- und Acrylpolymeren und Styrol-Acryl-Polymeren ausgewählt ist.

11. Zusammensetzung nach einem der vorhergehenden Ansprüche, **dadurch gekennzeichnet, daß** das Polymer unter den Polyetherpolyurethanen und den Polyesterpolyurethanen ausgewählt ist.

12. Zusammensetzung nach einem der vorhergehenden Ansprüche, **dadurch gekennzeichnet, daß** sie einen grenzflächenaktiven Stoff zum Emulgieren des fluorhaltigen Copolymers enthält.

13. Zusammensetzung nach einem der vorhergehenden Ansprüche, **dadurch gekennzeichnet, daß** das Färbemittel ein Farbstoff und/oder ein Pigment ist.

14. Zusammensetzung nach einem der vorhergehenden Ansprüche, **dadurch gekennzeichnet, daß** sie, angegeben in Gewichtsprozent, enthält:
- 0,005 bis 10 % fluorhaltiges Copolymer,
- 2 bis 60 % filmbildendes Material,
- 0 bis 10 % grenzflächenaktiven Stoff,
- 0 bis 10 % Hilfsstoff,
- 0 bis 25 % Färbemittel,
- 0 bis 5 % Verdickungsmittel und
- Wasser in einer ausreichenden Menge für 100 % Zusammensetzung.

15. Zusammensetzung nach einem der vorhergehenden Ansprüche, **dadurch gekennzeichnet, daß** das fluorhaltige Copolymer in einem Mengenanteil von 0,5 bis 5 Gew.-%, bezogen auf das Gesamtgewicht der Zusammensetzung, enthalten ist.

16. Zusammensetzung nach einem der vorhergehenden Ansprüche, **dadurch gekennzeichnet, daß** das filmbildende Material in einem Mengenanteil von 4 bis 30 Gew.-%, bezogen auf das Gesamtgewicht der Zusammensetzung, enthalten ist.

17. Zusammensetzung nach einem der vorhergehenden Ansprüche, **dadurch gekennzeichnet, daß** sie in Form eines Mascaras, eines Eyeliners oder eines Lacks vorliegt.

18. Zusammensetzung nach einem der Ansprüche 1 bis 16, **dadurch gekennzeichnet, daß** sie in Form eines Nagellacks vorliegt.

19. Verfahren zum Schminken und/oder zur Pflege der Haut, der Nägel, der Wimpern und/oder der Haare, **dadurch gekennzeichnet, daß** auf die Haut, die Nägel, die Wimpern und/oder die Haare eine Zusammensetzung, die wie in einem der Ansprüche 1 bis 18 definiert ist, aufgetragen wird.

20. Verwendung in einer kosmetischen Zusammensetzung, die ein in der Kosmetik verwendbares wäßriges Medium enthält, mindestens eines filmbildenden Materials und mindestens eines fluorhaltigen Polymers, die wie in einem der Ansprüche 1 bis 11 und 15, 16 definiert sind, die in dem wäßrigen Medium dispergiert sind, für die Herstellung eines Films, der kontinuierlich und homogen ist und/oder wasserfest ist und/oder eine gute Langzeitfestigkeit aufweist, wobei das wäßrige Medium nur Wasser, 0 bis 30 Gew.-% organisches, mit Wasser mischbares Lösemittel und gegebenenfalls einen Bestandteil enthält, der uner Färbemitteln, Hilfsstoffen, Verdikkungsmitteln, grenzflächenaktiven Stoffen ausgewählt ist.

## Claims

1. Film-forming cosmetic composition comprising an aqueous medium which may be used in cosmetics, **characterized in that** it comprises, dispersed in the said aqueous medium, at least one film-forming material and a fluorinated copolymer resulting from the copolymerization of at least one first fluorovinyl monomer of formula (B): 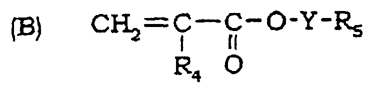 in which R₄ represents a hydrogen atom or a C₁ to C₄ alkyl radical; Y represents a C₁ to C₆ alkylene radical and R₅ represents a C₁ to C₂₀ alkyl radical all or some of the hydrogen atoms of which are replaced with fluorine atoms,
and of at least one second monomer of formula (A): 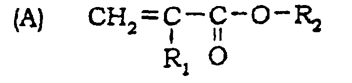 with R₁ representing a hydrogen atom or a C₁ to C₄ alkyl radical and R₂ representing a C₁ to C₆ alkyl radical, a C₂ to C₄ hydroxycarbon radical or a radical-(CH₂)ₚ-NH-R₃ with R₃ representing a C₁ to C₆ alkyl or a cycloalkyl and p being an integer ranging from 1 to 4,
the weight ratio of the first monomer relative to the second non-fluorinated monomer ranging from 10/90 to 90/10,
the aqueous medium consisting of water, from 0 to 30% by weight of water-miscible organic solvent, and optionally a dyestuff, an adjuvant, a thickener and/or a surfactant.

2. Composition according to Claim 1, **characterized in that** the adjuvant is chosen from UV screening agents, preserving agents, wetting agents, dispersants, antifoams, waxes, drying accelerators, spreading agents and compounds for improving the gloss or hardness of the film obtained after drying the composition.

3. Composition according to Claim 1 or 2, **characterized in that** it is free of organic solvent.

4. Composition according to any one of the preceding claims, **characterized in that** R₂ is a C₁ to C₆ alkyl radical.

5. Composition according to any one of the preceding claims, **characterized in that** the first monomer has the formula: 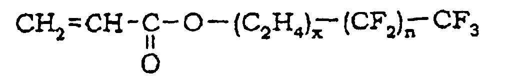 with n ranging from 4 to 16 and x being 1 or 2.

6. Composition according to any one of Claims 1 to 4, **characterized in that** the first monomer has the formula: 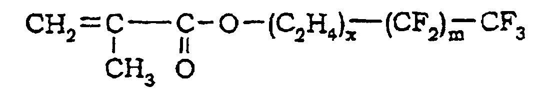 with m ranging from 4 to 16 and x being 1 or 2.

7. Composition according to any one of Claims 1 to 4, **characterized in that** the first monomer has the formula: 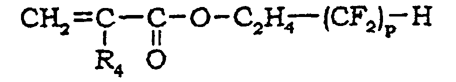 with R₄ = H or -CH₃ and p ranging from 6 to 16 and, for example, equal to 6 or 8.

8. Composition according to any one of the preceding claims, **characterized in that** the fluorinated copolymer represents from 0.005% to 10% by weight relative to the total weight of the composition.

9. Composition according to any one of the preceding claims, **characterized in that** the film-forming material is composed of at least one film-forming polymer.

10. Composition according to Claim 9, **characterized in that** the polymer is chosen from polyurethanes, vinyl and acrylic polymers, and styrene-acrylic polymers.

11. Composition according to any one of the preceding claims, **characterized in that** the polymer is chosen from polyether-polyurethanes and polyesterpolyurethanes.

12. Composition according to any one of the preceding claims, **characterized in that** it contains a surfactant to emulsify the fluorinated copolymer.

13. Composition according to any one of the preceding claims, **characterized in that** the dyestuff is a colorant and/or a pigment.

14. Composition according to any one of the preceding claims, **characterized in that** it contains, by weight:
- from 0.005% to 10% of fluorinated copolymer,
- from 2% to 60% of film-forming material,
- from 0% to 10% of surfactant,
- from 0% to 10% of an adjuvant,
- from 0% to 25% of dyestuff,
- from 0% to 5% of thickener, and
- water in an amount sufficient to make 100%.

15. Composition according to any one of the preceding claims, **characterized in that** the fluorinated copolymer represents from 0.5% to 5% by weight relative to the total weight of the composition.

16. Composition according to any one of the preceding claims, **characterized in that** the film-forming material represents from 4% to 30% by weight relative to the total weight of the composition.

17. Composition according to any one of the preceding claims, **characterized in that** it is in the form of a mascara, an eyeliner or a lacquer.

18. Composition according to any one of Claims 1 to 16, **characterized in that** it is in the form of a nail varnish.

19. Make-up process and/or cosmetic care process for the skin, the nails, the eyelashes and/or the hair, **characterized in that** a composition as defined according to any one of Claims 1 to 18 is applied to the skin, the nails, the eyelashes and/or the hair.

20. Use, in a cosmetic composition comprising an aqueous medium which may be used in cosmetics, of at least one film-forming material and of at least one fluorinated copolymer as defined according to any one of Claims 1 to 11, 15 and 16, dispersed in the said aqueous medium, to obtain a film which is homogeneous and continuous and/or water-resistant and/or which has good staying power over time, the aqueous medium containing only water, from 0 to 30% by weight of water-miscible organic solvent, and optionally an ingredient chosen from dyestuffs, adjuvants, thickeners and surfactants.
